# EUROPÄISCHE PATENTSCHRIFT

(11) **EP 0 746 596 B1**
(45) Veröffentlichungstag und Bekanntmachung des Hinweises auf die Patenterteilung: **13.01.1999**
(21) Anmeldenummer: 95910410.0
(22) Anmeldetag: 16.02.1995
(51) Int. Cl.: C10G 9/16

(54) **VERFAHREN ZUR HERSTELLUNG VON THERMISCH GECRACKTEN PRODUKTEN AUS KOHLENWASSERSTOFFEN**
PROCESS FOR PRODUCING THERMALLY CRACKED PRODUCTS FROM HYDROCARBONS
PROCEDE DE PRODUCTION PAR CRAQUAGE THERMIQUE DE PRODUITS D'HYDROCARBURES

(30) Priorität: 21.02.1994 DE 4405883
(43) Veröffentlichungstag der Anmeldung: 11.12.1996
(73) Patentinhaber: MANNESMANN Aktiengesellschaft, 40213 Düsseldorf (DE); KTI GROUP B.V., NL-2700 AB Zoetermeer (NL)
(72) Erfinder: ZIMMERMANN, Gerhard, D-04299 Leipzig (DE); ZYCHLINSKI, Wolfang, D-04318 Leipzig (DE)
(74) Vertreter: Meissner, Peter E., Dipl.-Ing.
(86) Internationale Anmeldenummer: DE9500233
(87) Internationale Veröffentlichungsnummer: WO9522587

(56) Entgegenhaltungen:
- EP-A- 0 241 020
- US-A- 4 024 049
- US-A- 4 116 812
- US-A- 4 756 819

## Beschreibung

Die Erfindung betrifft ein Verfahren zur Herstellung von thermisch gecrackten Produkten aus Kohlenwasserstoffen unter gleichzeitiger Reduzierung der Koksablagerungen auf den Wärmeaustauschflächen.

Zur Herstellung von Ethylen und anderen Alkenen werden Kohlenwasserstoffe oder Kohlenwasserstoffgemische in von außen beheizten Reaktoren aus metallischen Werkstoffen thermisch gecrackt. Die Cracköfen bestehen vorzugsweise aus hochtemperaturbeständigen chrom- und nickelhaltigen Rohren.

Die Wirksamkeit der Rohrreaktoren hängt stark davon ab, ob sich im Betrieb auf den Innenoberflächen der Reaktorrohre koksartige Ablagerungen bilden, die den Wärmeübergang behindern. Bei den heute verwendeten Reaktoren ist das regelmäßig der Fall. Nach Betriebszeiten zwischen einer Woche und drei Monaten erreichen die auf den mit den Kohlenwasserstoffen in Kontakt tretenden Innenseiten der Reaktoren gebildeten Ablagerungen ein solches Ausmaß, daß die dadurch bedingten Leistungsminderungen zu einer Außerbetriebnahme und zu aufwendigen Reinigungsprozeduren zwingen. Die Entfernung der koksartigen Ablagerungen erfolgt meist in der Weise, daß sie durch ein Gemisch aus heißem Wasserdampf und Luft vergast werden, wobei die metallischen Oberflächen wieder freigelegt werden und der angestrebte Wärmefluß wieder gewährleistet wird. Trotz gründlicher Entfernung des abgelagerten Kokses können die erneut gebildeten Ablagerungen bereits nach relativ kurzen Betriebszeiten wieder zur Außerbetriebnahme und Entkokung zwingen. Da die angewendeten oxidativen Enkokungsprozeduren eine Hochtemperaturkorrosion der Rohre bewirken, in deren Ergebnis Reaktionsprodukte entstehen, welche die Koksbildung katalysieren, wird die Ablagerung koksartiger Produkte nach jeder Entkokung beschleunigt, so daß die Betriebszeiten zwischen den jeweiligen Entkokungsprozeduren mit der Anzahl an Dekokungszyklen kürzer werden. Das ist aus technischer wie aus wirtschaftlicher sicht gleichermaßen unerwünscht, weil auf diese Weise lange stationäre Betriebszustände verhindert werden, die effektive Anlagennutzung verringert wird und die Kosten für die Reinigungsprozedur häufiger anfallen. Man ist deshalb seit Jahren bemüht, Lösungen zu finden, die einer raschen Verkokung der Innenoberflächen von Rohrreaktoren entgegenwirken. Zum Erreichen dieses Zieles wurde in einigen Anlagen eine Behandlung mit Alkyl-Ammoniumsulfonaten angewendet, die jedoch nicht zur Verminderung der Koksbildung führte. In der US-A-4.105.540 wird die Zugabe kleiner Mengen von Phosphor- und Phosphorigsäureestern und in der US-A-4.551.227 die Zugabe einer Kombination von Zinn- und Phosphor-, Phosphor- und Antimon- oder Zinn-, Antimon- und Phosphorverbindungen als Antifoulants beschrieben. In der US-A-3.647.677 und US-A-3.531.394 werden als Antifoulants elementarer Phosphor in Raffinerieanlagen bzw. Phosphorverbindungen in Steamcracking-Anlagen vorgeschlagen. Analoges gilt für die US-A-4.835.332 (Triphenylphosphin), US-A-4.900.426 (Triphenylphosphinoxid) und US-A-4.842.716, in dem die Zugabe einer Kombination aus einem Phosphorantifoulant und einem die Filmbildung begünstigenden Inhibitor (Imidazoline) beschrieben wird. Schließlich werden auch gas- oder dampfförmige schwefelhaltige (Boene, K.: Oil Gas J. 81 (1983) 93) und stickstoffhaltige Verbindungen als Additive mit Antifoulingwirkung beschrieben. All diese Additive bewirken offenbar eine begrenzte Verminderung der Verkokungstendenz ohne jedoch die Aufkohlung der Werkstoffoberflächenschichten zu hemmen, die als der Beginn des Ablaufes einer unerwünschten Reaktionskaskade aufzufassen ist. Da am Ende dieses Prozesses die Entstehung katalytisch aktiver Zentren steht, wird durch die Anwendung dieser Antifoulants den Symptomen, nicht aber den Ursachen der Koksbildung begegnet.

Die Aufkohlung von metallischen Werkstoffoberflächen wird bekanntermaßen durch relativ hohe Siliziumgehalte in diesen Oberflächenschichten gehemmt (Norton, J.F., Barnes, J.: Corrosion in Fuel systems, The Electrochemical Soc., Pennington, NJ, 1983, 277), wobei diese Hemmung unter den Bedingungen des thermischen Crackens offenbar durch Abreicherung der Siliziumspezies allmählich zurückgeht.

Außerdem ist bekannt, daß Antifoulants, die aus Kombinationen von anorganischen und/oder organischen Si- und Sn-und/oder Sb-Verbindungen bestehen, eine kokshemmende Wirkung bei der Herstellung von Crackprodukten entfalten sollen (EP-A-0241020).

Der Erfindung liegt daher die Aufgabe zugrunde, eine Lösung vorzuschlagen, mit der die Verkokung wesentlich verringert und gleichzeitig die Langzeitpassivität von Hochtemperaturstählen mit vergleichsweise hohen Siliziumgehalten in der Oberflächenschicht über lange Betriebsperioden erhalten wird.

Erfindungsgemäß besteht das Verfahren zur Herstellung von thermisch gecrackten Produkten aus Kohlenwasserstoffen unter gleichzeitiger Reduzierung der Koksablagerungen auf den Wärmeaustauschflächen darin, daß dem zu crackenden Einsatzprodukt vor dem Erreichen der Cracktemperatur 20 bis 1000 ppm einer Additivformulierung hinzugesetzt werden, die ausgewählt ist unter
(1) einer oder mehreren Silizium und Schwefel enthaltenden flüchtigen organischen Verbindungen;
(2) einem Gemisch von organischen siliziumhaltigen flüchtigen Verbindungen und organischen schwefelhaltigen flüchtigen Verbindungen;
(3) einem Gemisch von Silizium und Schwefel enthaltenen flüchtigen organischen Verbindungen und flüchtigen siliziumhaltigen und/oder flüchtigen schwefelhaltigen organischen Verbindungen; oder
(4) bei schwefelreichen Einsatzprodukten mit >100 ppm Schwefel, bei denen die Schwefelkomponente unter Crackbedingungen flüchtig wird, nur siliziumhaltigen flüchtigen organischen Verbindungen, höchstens jedoch 300 ppm der reinen siliziumhaltigen Verbindung;
wobei in jedem Fall das Atomverhältnis von Silizium und Schwefel jeweils 5:1 bis 1:1 beträgt;
und das Gemisch mit den Wärmeaustauschflächen, die Temperaturen im Bereich von 400 bis 1000 'C aufweisen, in Kontakt gebracht wird.

Dabei beträgt die Temperatur 700 bis 1000 'C, wenn die Wärmeaustauschfläche die metallische Innenwand eines Rohrreaktors ist. Die Temperatur beträgt 400 bis 750 'C, wenn die Wärmeaustauschfläche die metallische Fläche eines Wärmeaustauschers ist, der beispielsweise dem Rohrreaktor nachgeschaltet ist, wobei die Temperatur am Eingang der Wärmeaustauschers (Aufprallplatte) auch örtlich über 800 'C liegen kann, z.B. 875 'C im Einzelfall betragen kann.

Die Silizium und/oder Schwefel enthaltende Verbindung ist vorzugsweise ausgewählt aus der Gruppe, die aus Trimethylsilylmercaptan, Dimethylsulfid, Tetramethylsilan und Bis-trimethylsilylsulfid und deren Gemischen. Es können aber auch andere flüchtige Verbindungen eingesetzt werden, sofern damit das Ziel der vorliegenden Erfindung erreicht wird.

Wenn ein besonders schwefelreiches Einsatzprodukt vorliegt, kann gegebenenfalls auf den Einsatz der schwefelhaltigen flüchtigen Verbindung verzichtet werden und nur die siliziumreiche flüchtige Verbindung hinzugesetzt werden, sofern sich das erfindungsgemäße Atomverhältnis im Bereich von 5:1 bis 1:1 ergibt und die Schwefelkomponente im Einsatzprodukt unter Crackbedingungen flüchtig wird. Diese Ausführungsform der Erfindung kann z.B. bei >100 ppm Schwefel im Einsatzprodukt ausgeführt werden, wobei die siliziumreiche Verbindung jedoch höchstens mit 300 ppm hinzugesetzt werden sollte.

Der Erfindung liegt die überraschende Erkenntnis zugrunde, daß eine Zugabe von flüchtigen organischen Verbindungen, die entweder Silizium und Schwefel enthalten oder ein Gemisch von Silizium und Schwefel enthaltenden Verbindungen darstellen, zu Pyrolyseeinsatzprodukten unter Pyrolysebedingungen eine Wirkung entfalten, die einerseits offenbar geeignet ist, den Siliziumspiegel in der Oberflächenschicht von siliziumhaltigen metallischen Werkstoffen, der während des thermischen Crackens abgebaut wird, ständig wieder aufzufrischen und damit die Koksbildung ad hoc auf einem niedrigen Niveau zu halten, und andererseits die Verkokung an den Wärmeaustauschflächen von Reaktorrohren, die aus relativ siliziumarmen Stählen gefertigt sind, signifikant zu vermindern.

Ausschließlich Silizium enthaltende organische Verbindungen zeigen keine inhibierende Wirkung der Koksbildung, ausschließlich Schwefel enthaltende Verbindungen sind nicht in der Lage, der als Ursache für die Bildung katalytisch aktiver Zentren verantwortlichen Aufkohlung der Werkstoffoberflächen entgegenzuwirken.

Um beide Wirkungen zu erzielen, insbesondere aber die Langzeitpassivität der mit Crackprodukten in Berührung kommenden Wärmetauschflächen von Rohrreaktoren zu erhalten, sieht die Erfindung vor, die thermisch zu crackenden Einsatzkohlenwasserstoffe vor Beginn der eigentlichen Crackreaktionen mit mindestens 20 ppm und vorzugsweise 50 bis 400 ppm, insbesondere 50 bis 200 ppm einer Silizium und Schwefel enthaltenden flüchtigen organischen verbindung oder mit einem Gemisch von flüchtigen Silizium- und Schwefelverbindungen zu versetzen. Das geschieht zweckmäßigerweise so, daß eine Lösung dieser Verbindungen in einer geeigneten Kohlenwasserstofffraktion hergestellt und diese Lösung kontinuierlich zu der zu crackenden Kohlenwasserstofffraktion zudosiert wird. Auf diese Weise werden die niedrigen Verkokungsgeschwindigkeiten, die für die Innenoberflächen von Crackrohren aus silizium-haltigen Stählen typisch sind, über lange Betriebsperioden aufrecht erhalten.

Es hat sich als günstig herausgestellt, die thermisch zu crackenden Kohlenwasserstoffe vor dem Erreichen der eigentlichen Cracktemperatur mit einem Additiv zu versetzen, das entweder aus einer flüchtigen Verbindung besteht, die Silizium und Schwefel im Atomverhältnis 5:1 bis 1:1 enthält, oder aus einem Gemisch aus einer silizium- und einer schwefelhaltigen Verbindung besteht, in der das gleiche Atomverhältnis vorliegt.

Das gecrackte Endprodukt ist vorzugsweise ausgewählt aus der Gruppe, die aus Alkenen wie Ethylen, Propylen, Butadien und anderen niederen Olefinen und Diolefinen besteht.

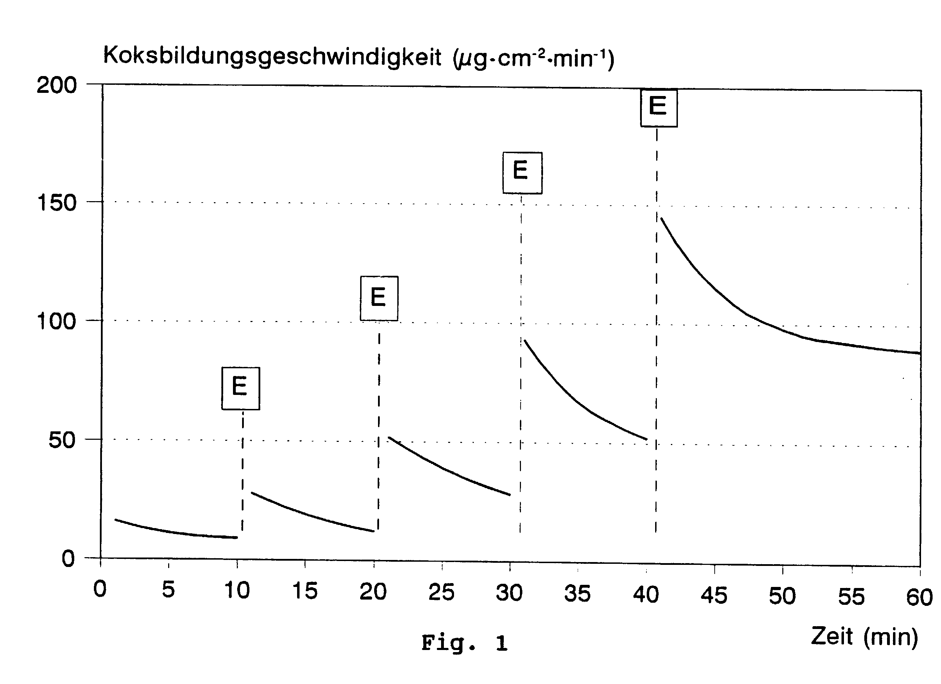

Nachfolgend wird die Erfindung anhand mehrerer Vergleichsbeispiele und erfindungsgemäßer Ausführungsbeispiele näher erläutert. Die Figuren 1 bis 7 beschreiben die Abhängigkeiten der Kolksbildungsgeschwindigkeiten an voraktivierten und solchen Probekörpern aus Chrom-Nickel-Stahl, die durch eine spezielle thermische Vorbehandlung mit Silizium und Schwefel enthaltenden Verbindungen eine verringerte Neigung zum Aufwachsen von Koks zeigen, von der Versuchszeit bei der Pyrolyse von n-Heptan in Stickstoff und in Wasserdampf als Verdünnungsmittel bei Zusatz bekannter und erfindungsgemäßer Kolksbildunginhibitoren.
Fig. 1: - Abhängigkeit der Koksbildungsgeschwindigkeit an einem voraktivierten Probekörper aus Chrom-Nickel-Stahl (X8CrNiTi18.10) von der Versuchszeit und der Anzahl der Entkokungen mittels Luft (E) bei der n-Heptanpyrolyse in Stickstoff (T_{R} = 715 °C, τ = 1 s)
Fig. 2: - Abhängigkeit der Koksbildungsgeschwindigkeit an einem voraktiverten Probekörper aus Chrom-Nickel-Stahl (X8CrNiTi18.10) von der Versuchszeit bei der n-Heptanpyrolyse ohne und mit Zusatz von 85 ppm Dimethyldisulfid (DMDS) (T_{R} = 715 °C, τ = 1 s)
Fig. 3. - Abhängigkeit der Koksbildungsgeschwindigkeit an einem voraktivierten Probekörper aus Chrom-Nickel-Stahl (X8CrNiTi18.10) von der Versuchszeit bei der Pyrolyse von reinem n-Heptan und einem 1000 ppm Triphenylphosphinoxid (TPPO) enthaltenden n-Heptan (T_{R} = 715 °C, τ = 1 s)
Fig. 4. - Abhängigkeit der Koksbildungsgeschwindigkeit an einem voraktivierten Probekörper aus Chrom-Nickel-Stahl (X8C-rNiTi18.10) von der Versuchszeit bei der Pyrolyse von n-Heptan ohne und mit einem Zusatz von 100 ppm Tetramethylsilan (TMSi) (T_{R} = 715 °C, τ = 1 s).
Fig. 5. - Abhängigkeit der Koksbildungsgeschwindigkeit an einem mit Trimethylsilylmethylmercaptan thermisch bei 880 °C vorbehandelten Probekörper aus Chrom-Nickel-Stahl (X8CrNiTi18.10) von der Versuchszeit bei der Pyrolyse von reinem n-Heptan (T_{R} = 715 °C, τ = 1s).
Fig. 6. - Abhängigkeit der Koksbildungsgeschwindigkeit an einem mit Trimethylsilylmethylmercaptan thermisch bei 880 °C vorbehandelten Probekörper aus unbenutztem Chrom-Nickel-Stahl (X8CrNiTi18.10) von der Versuchszeit bei der n-Heptanpyrolyse in Gegenwart von 100 ppm Trimethylsilylmethylmercaptan als Additiv.
Fig. 7. - Beeinflussung der Koksbildungsgeschwindigkeit an voraktivierten Probekörpern aus Chrom-Nickel-Stahl (X8CrNiTi18.10) bei der Pyrolyse von n-Heptan in Abwesenheit (Kurve A, erster Abschnitt) und Gegenwart (Kurve B und zweiter Abschnitt Kurve A) von 100 ppm Trimethylsilylmethylmercaptan (TMSMM).

### Beispiel 1 (Vergleichsbeispiel)

In einer elektrisch beheizten Laborpyrolyseapparatur aus Quarz (vertikal angeordneter Laborrohrreaktor, (dᵢ = 20 mm; V_{R} = 13 ml)), in dessen Inneren sich ein Werkstoffprobekörper befindet, der über einen dünnen Platindraht mit einer Mikro-Thermowaage verbunden ist [vg. J. Anal. Appl. Pyrolysis 27 (1993) 45]) wurde n-Heptan unter Normaldruck in Stickstoff oder Wasserdampf als Verdünnungsmittel bei Temperaturen zwischen 700 und 800 °C und Verweilzeiten ( τ ) um etwa 1 s pyrolysiert. Der n-Heptandurchsatz betrug 14 ml/h, der Verdünnungsmitteldurchsatz 5 l/h. Das Gewichtsverhältnis Ethylen zu Propylen im Spaltgas lag unter den angegebenen Bedingungen bei 2,0 bis 2,7. Die Probekörper hatten eine Abmessung von 30 x 7 x 1 mm.

Die Gewichtsveränderung des Probekörpers durch Koks, der auf der Oberfläche des Probekörpers aufwächst, wurde kontinuierlich gemessen und die Koksbildungsgeschwindigkeit (µg/cm^{2 ·} min) in Abhängigkeit von der Versuchszeit ermittelt. Um die Wirkung von Koksbildungsinhibitoren zuverlässig beurteilen zu können, die dem n-Heptan vor Eintritt in die Pyrolyseapparatur in Anteilen von 10 bis 250 ppm zugemischt wurden, wurden bei Einsatz von reinem n-Heptan Kolksbildungsgeschwindigkeiten im Bereich von 50 bis 300 µg/cm^{2 ·}min eingestellt. Das machte eine Erhöhung der katalytischen Aktivität der Werkstoffoberflächen durch mehrmaliges Verkoken und Entkokung mittels Luft erforderlich (Voraktivierung der Probekörper, um eine katalytische Aktivität zu erreichen, wie sie in industriellen Anlagen erst nach mehrmaligem Entkoken vorliegt). In Fig. 1 ist ein typischer Verlauf der Koksbildungsgeschwindigkeit auf der Oberfläche eines Chrom-Nickel-Stahl-Probekörpers von der Versuchszeit bei der Pyrolyse von reinem n-Heptan in Stickstoff bei 715 °C für fünf aufeinanderfolgende Verkokungs-Entkokungs-Zyklen dargestellt.

### Beispiel 2 (Vergleichsbeispiel)

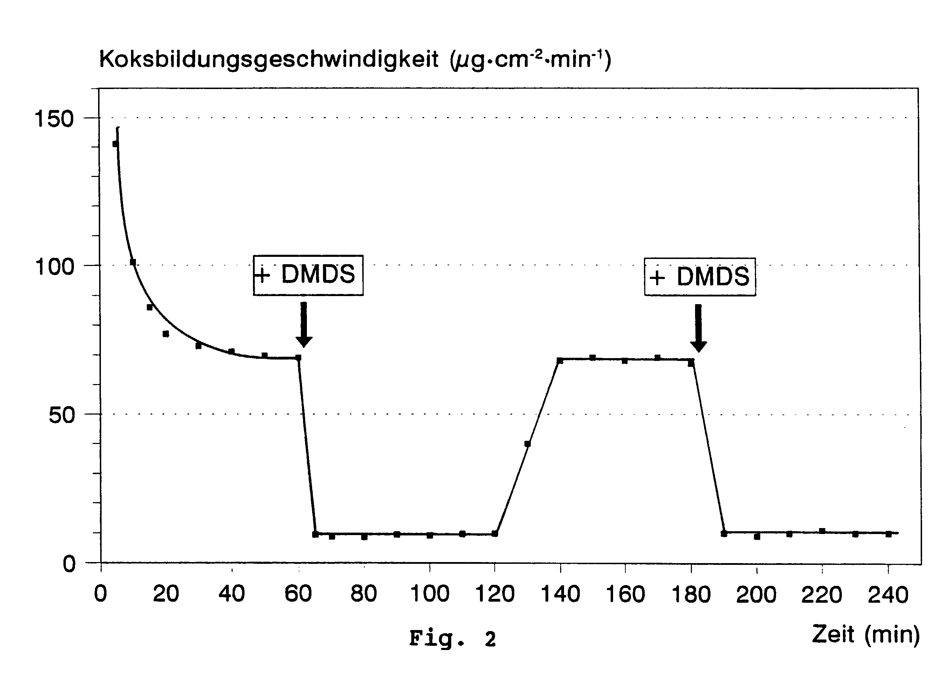

In der gleichen Apparatur wie im Beispiel 1 beschrieben, wurde zunächst reines n-Heptan in Gegenwart eines voraktivierten Probekörpers aus Chrom-Nickel-Stahl (X8CrNiTi18.10) bei 715 °C in Gegenwart von Stickstoff pyrolysiert und die Koksbildungsgeschwindigkeit verfolgt. Nach 60 Minuten wurde die Zuführung von reinem n-Heptan unterbrochen und auf ein Gemisch als Einsatzprodukt umgeschaltet, in dem 85 ppm Dimethyldisulfid (DMDS) in n-Heptan gelöst waren. Wie der Fig. 2 zu entnehmen ist, trat nach dem Wechsel des Einsatzproduktes eine drastische Verringerung der Koksbildungsgeschwindigkeit ein, die bei der Umstellung auf reines n-Heptan wieder aufgehoben wurde.

### Beispiel 3 (Vergleichsbeispiel)

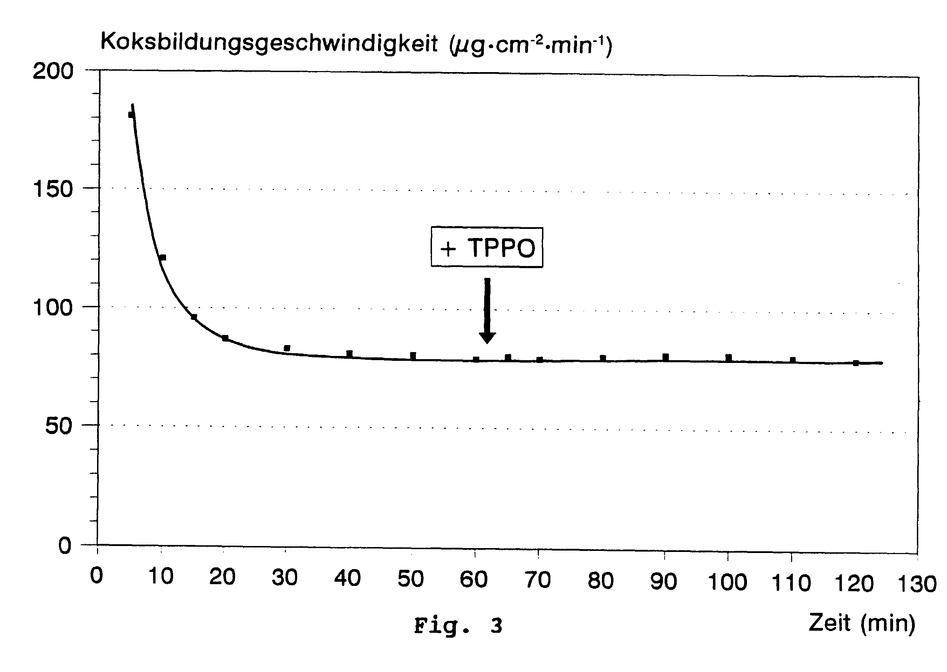

In der gleichen Apparatur wie in Beispiel 1 beschrieben und unter den gleichen Bedingungen wie im Beispiel 2 angeführt wurde die Wirkung von Triphenylphosphinoxid (TPPO) [siehe US-P. 4.900.426 vom 13.02.1990] an Stelle von Dimethyldisulfid untersucht. Die Ergebnisse sind in Fig. 3 dargestellt. Danach tritt beim Umschalten von reinem n-Heptan auf ein Gemisch von n-Heptan, das mit 1000 ppm Triphenylphosphinoxid versetzt war, unter den angewendeten Versuchsbedingungen keine signifikante Beeinflussung der Koksbildung ein.

### Beispiel 4 ( vergleichsbeispiel)

In der gleichen Apparatur wie im Beispiel 1 beschrieben und unter den in den Beispielen 2 und 3 angegebenen Bedingungen wurde der Einfluß von Tetramethylsilan (TMSi) auf die Koksbildung untersucht.

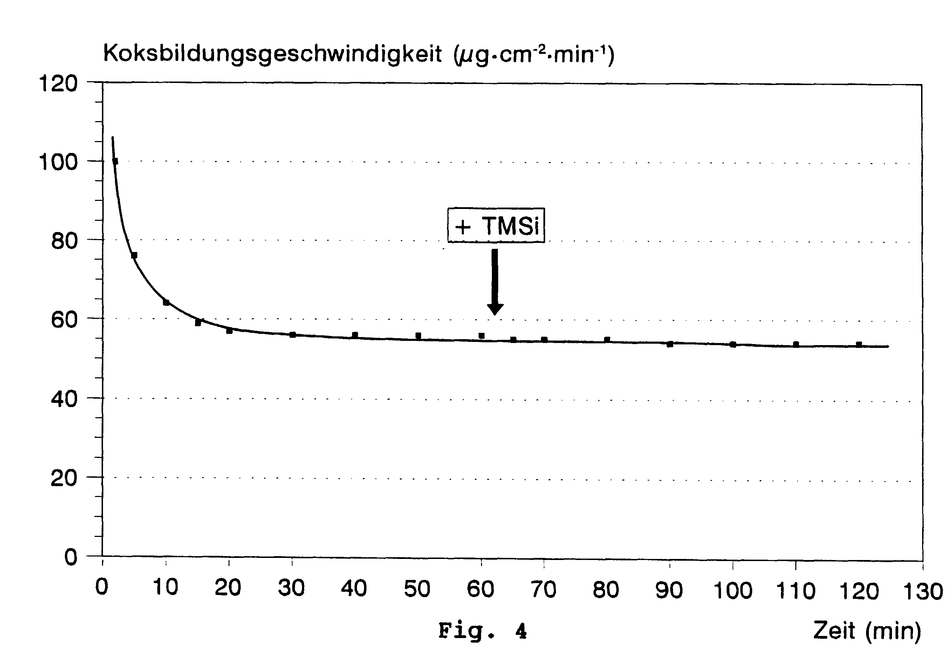

Die Ergebnisse sind in Fig. 4 dargestellt. Danach tritt beim Umschalten von reinem n-Heptan auf ein mit 100 ppm Tetramethylsilan versetztes n-Heptan keine signifikante Absenkung der Koksbildungsgeschwindigkeit ein.

### Beispiel 5 (Vergleichsbeispiel)

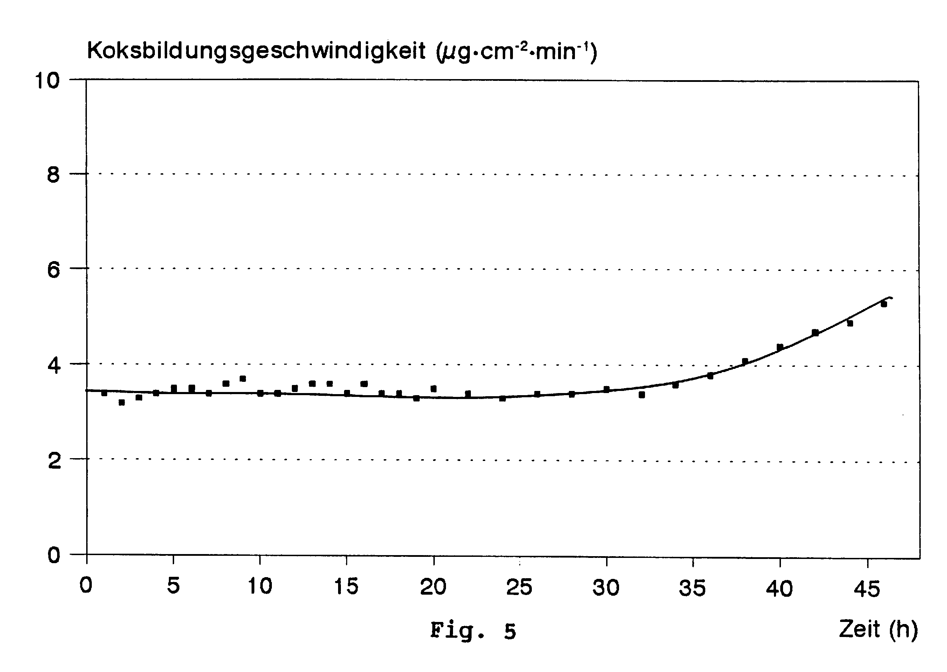

In der gleichen Apparatur wie in Beispiel 1 beschrieben wurde ein voraktivierter Probekörper aus Chrom-Nickel-Stahl (X8CrNiTi18.10) zunächst mit 3 l eines 0,005 mol Trimethylsilylmethylmercaptan enthaltenden äquimolaren Gemisches aus Wasserstoff und Methan über einen Zeitraum von 60 Minuten bei 880 °C nach einem gleichfalls von den vorliegenden Erfindern angemeldeten Verfahren thermisch vorbehandelt. Die Apparatur wurde mit Stickstoff gespült und danach die Koksbildungsgeschwindigkeit bei der Pyrolyse von reinem n-Heptan in Stickstoff als Verdünnungsmittel (n_{Heptan} : n_{N2} = 0,5) über eine Versuchszeit von 45 Stunden ermittelt. Das Ergebnis ist in Fig. 5 dargestellt. Es zeigt, daß die für derartige thermische vorbehandlungen typischen niedrigen Koksbildungsgeschwindigkeiten nach etwa 35 Stunden ansteigen.

### Beispiel 6 (erfindungsgemäßes Ausführungsbeispiel)

In der gleichen Apparatur wie in Beispiel 1 beschrieben und unter den analogen äußeren Bedingungen, die den Beispielen 2 bis 4 zugrundeliegen, wurde in einem Langzeitversuch der Einfluß von Trimethylsilylmethylmercaptan auf die Koksbildungsgeschwindigkeit an nach Beispiel 5 mit Trimethylsilylmethylmercaptan thermisch vorbehandelten Probekörpern aus Chrom-Nickel-Stahl (X8CrNiTi18.10) untersucht.

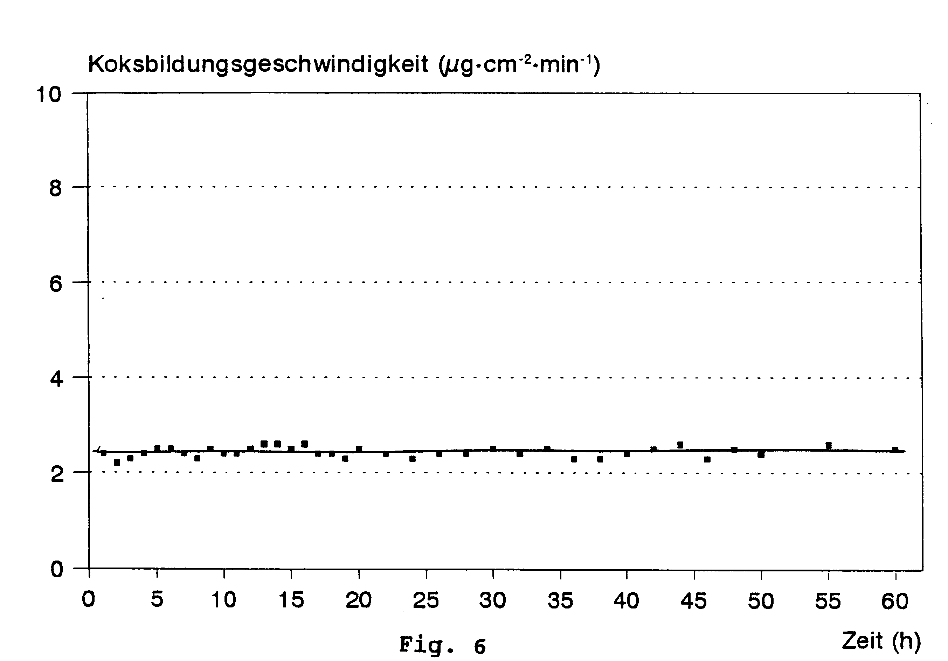

Die Ergebnisse sind in Fig. 6 wiedergegeben. Ein Vergleich zeigt, daß die an dem nach Beispiel 5 mit Trimethylsilylmethylmercaptan thermisch vorbehandelten Probekörper ohnehin geringe Koksbildungsgeschwindigkeit durch Zusatz von 100 ppm dieser Verbindung zum n-Heptan weiter verringert werden kann und daß darüber hinaus der ohne Zugabe von Trimethylsilylmethylmercaptan nach etwa 35 Versuchsstunden einsetzende Anstieg der Koksbildungsgeschwindigkeit durch diese zusätzliche kontinuierliche Zugabe von Trimethylsilylmethylmercaptan als Additiv zum Pyrolyseeinsatzprodukt vermieden werden kann.

### Beispiel 7 (erfindungsgemäßes Ausführungsbeispiel)

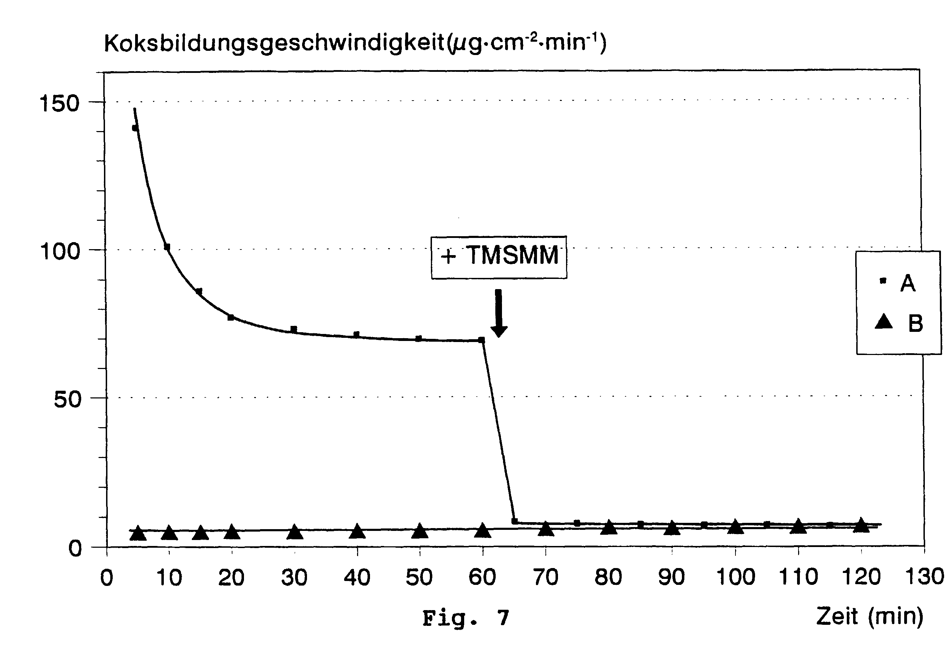

In der gleichen Apparatur wie in Beispiel 1 beschrieben und unter den analogen äußeren Bedingungen wie in den Beispielen 2 bis 4 angegeben wurde der Einfluß von 100 ppm Trimethylsilylmethylmercaptan (TMSMM) auf die Koksbildungsgeschwindigkeit bei der n-Heptanpyrolyse untersucht. Dabei wurde einerseits der Verlauf der Koksbildungsgeschwindigkeit beim Umschalten von reinem n-Heptan auf ein mit 100 ppm Trimethylsilylmethylmercaptan versetztes n-Heptan verfolgt (Fig. 7, Kurve A) und andererseits die Koksbildungsgeschwindigkeit an einem frisch entkokten Probekörper bei sofortiger Dosierung des mit 100 ppm Trimethylsilylmethylmercaptan versetzten n-Heptan ermittelt (Fig. 7, Kurve B). In Fig. 7 sind die erhaltenen Ergebnisse dargestellt. Es ist ersichtlich, daß die erfindungsgemäße Verwendung von Silizium und Schwefel enthaltenden Verbindungen als Additiv bei kontinuierlicher Zugabe zu dem als Pyrolyseeinsatzprodukt verwendeten n-Heptan zu einer signifikanten Absenkung der Koksbildungsgeschwindigkeit führt.

### Beispiel 8 (erfindungsgemäßes Ausführungsbeispiel)

In der gleichen Apparatur wie in Beispiel 1 beschrieben, wurden voraktivierte Probekörper aus Chrom-Nickel-Stahl (X8CrNiTi18.10) unter den im Beispiel 5 angeführten Bedingungen mit Trimethylsilylmethylmercaptan thermisch vorbehandelt und anschließend die Koksbildungsgeschwindigkeiten an diesen Probekörpern bei der n-Heptanpyrolyse nach den Beispielen 2 bis 4 in Abhängigkeit von unterschiedlichen Mengen Silizium und Schwefel enthaltenden Additiven ermittelt.

Der Gesamtanteil an den erfindungsgemäßen Additivformulierungen, die dem n-Heptan zudosiert wurden, betrug in jedem Falle 120 ppm. Die in Abhängigkeit von den Versuchszeiten gemessenen Koksbildungsgeschwindigkeiten sind in Tab. 1 zusammengestellt. Sie weisen aus, daß an Stelle von gleichzeitig Silizium und Schwefel enthaltenden Verbindungen auch Gemische aus Silizium- und Schwefelverbindungen als Koksbildungsinhibitoren die gleiche Wirkung erzielen, wobei das Atomverhältnis von Silizium zu Schwefel im Bereich zwischen 1:1 und 5:1 schwanken kann.

### Beispiel 9 (erfindungsgemäßes Beispiel)

In der gleichen Apparatur wie in Beispiel 1 beschrieben, wurde ein Probekörper aus Chrom-Nickel-Stahl (X8CrNiTi18.10), wie in Beispiel 5 erläutert, bei 880 °C mit Trimethylsilylmethylmercaptan thermisch vorbehandelt und danach die Koksbildungsgeschwindigkeiten an diesem Probekörper bei der Pyrolyse von n-Heptan, dem 100 ppm Trimethylsilylmethylmercaptan zugesetzt waren, in Gegenwart von Wasserdampf als Verdünnungsmittel (n_{Heptan} : n_{H2O(D)} = 0,5) verfolgt. Die Ergebnisse sind in Tab. 2 zusammengestellt. Sie weisen aus, daß die erfindungsgemäßen Additivformulierungen auch in Gegenwart von Wasserdampf als Verdünnungsmittel wirksam sind.

### Beispiel 10 (Vergleich und Erfindung)

In einer Laborpyrolyseapparatur gemäß Beispiel 1 wurden die Koksbildungsgeschwindigkeiten an fünf Probekörpern aus Chrom-Nickel-Stahl (X8CrNiTi18.10) untersucht, die analog Beispiel 5 bei 880 °C mit Trimethylsilylmethylmercaptan in einem äquimolaren Gemisch aus Wasserstoff und Methan 60 Minuten lang thermisch vorbehandelt waren. Als Pyrolyseeinsatzprodukte (PEP) wurden verwendet
- PEP 1: reines n-Heptan
- PEP 2: 100 ppm Tetramethylsilan in n-Heptan
- PEP 3: 100 ppm Dimethylsulfid in n-Heptan
- PEP 4: 100 ppm Trimethylsilylmethylmercaptan in n-Heptan
- PEP 5: 100 ppm eines äquimolaren Gemisches aus Tetramethyl silan und Dimethylsulfid in n-Heptan.

Die Pyrolysetemperatur betrug 715 °C, die Verweilzeit 1 s. Als Verdünnungsmittel diente Stickstoff (n_{Heptan} : n_{N2} = 0,5). Die über eine Versuchszeit von 45 Stunden ermittelten Koksbildungsgeschwindigkeiten sind in Tab. 3 zusammengestellt.
Von den fünf Pyrolyseeinsatzprodukten sind nur die Produkte PEP 4 und PEP 5 erfindungsgemäß. Ein Vergleich zeigt, daß nur bei Anwendung der erfindungsgemäß zusammengesetzten Proben die niedrigen Koksbildungsgeschwindigkeiten über lange Versuchszeiten aufrecht zu erhalten sind.

**Tabelle 1:**

| **Abhängigkeit der Koksbildungsgeschwindigkeit r bei der Pyrolyse von n-Heptan im Stickstoffstrom (715 °C, 1s) vom Atomverhältnis Silizium und Schwefel verschiedener Additivformulierungen, die dem n-Heptan zugesetzt wurden** | | | | | | |
|---|---|---|---|---|---|---|
| Atomverhältnis Si:S | a) 1:1 | b) 1:1 | c) 2:1 | d) 2:1 | e) 3:1 | f) 5:1 |
| Versuchszeit [min] | r [µg^{·}cm^{-2·}min⁻¹] | | | | | |
| 10 | 4,5 | 4,5 | 6,0 | 5,7 | 7,4 | 20 |
| 30 | 5,0 | 5,1 | 5,2 | 5,0 | 7,6 | 19 |
| 50 | 4,8 | 4,7 | 5,8 | 6,1 | 7,5 | 21 |
| 70 | 5,1 | 4,6 | 5,8 | 6,0 | 7,8 | 18 |
| 90 | 4,5 | 4,5 | 6,1 | 6,2 | 7,6 | 22 |
| 100 | 4,9 | 4,8 | 6,2 | 6,0 | 7,7 | 22 |

Als Additivkomponenten verwendete Si, S-Verbindungen:
a) Trimethylsilylmethylmercaptan
b) 1:1-Gemisch aus Tetramethylsilan und Dimethylsulfid
c) Bis-trimethylsilylsulfid
d) 2:1-Gemisch aus Tetramethylsilan und Dimethylsulfid
e) 3:1-Gemisch aus Tetramethylsilan und Dimethylsulfid
f) 5:1-Gemisch aus Tetramethylsilan und Dimethylsulfid

**Tabelle 2:**

| **Abhängigkeit des Einflusses von 100 ppm Trimethylsilylmethylmercaptan auf die Koksbildungsgeschwindigkeit r von der Versuchszeit bei der Pyrolyse von n-Heptan in Gegenwart von Wasserdampf (715 °C, 1s)** | |
|---|---|
| Versuchszeit [h] | r [_g^{·}cm^{-2·}min⁻¹] |
| 0,5 | 4 |
| 1 | 3 |
| 2 | 5 |
| 5 | 4 |
| 8 | 4 |
| 12 | 7 |

**Tabelle 3:**

| **Abhängigkeit der Koksbildungsgeschwindigkeit r an thermisch speziell vorbehandelten Probekörpern aus Chrom-Nickel-Stahl (X8CrNiTi18.10) von der Art der Additivzusätze und von der Versuchszeit** | | | | | |
|---|---|---|---|---|---|
| Edukt | PEP 1 | PEP 2 | PEP 3 | PEP 4 | PEP 5 |
| Versuchszeit [h] | r [µg^{·}cm^{-2·}min⁻¹] | | | | |
| 1 | 60 | 58 | 3 | 5 | 3 |
| 3 | 58 | 57 | 3 | 4,5 | 5 |
| 5 | 57 | 56 | 4 | 4,5 | 4 |
| 10 | 55 | 54 | 12 | 5,5 | 7 |
| 20 | 52 | 51 | 17 | 7 | 7 |
| 30 | 51 | 56 | 28 | 6 | 9 |
| 40 | 47 | 49 | 27 | 9 | 8,5 |
| 50 | 49 | 49 | 36 | 8 | 10 |

## Patentansprüche

1. Verfahren zur Herstellung von thermisch gecrackten Produkten aus Kohlenwasserstoffen unter gleichzeitiger Reduzierung der Koksablagerungen auf den Wärmeaustauschflächen, dadurch gekennzeichnet, daß dem zu crackenden Einsatzprodukt 20 bis 1000 ppm einer Additivformulierung hinzugesetzt werden, die ausgewählt ist unter
(1) einer oder mehreren Silizium und Schwefel enthaltenden flüchtigen organischen Verbindungen;
(2) einem Gemisch von organischen siliziumhaltigen flüchtigen Verbindungen und organischen schwefelhaltigen flüchtigen Verbindungen;
(3) einem Gemisch von silizium und Schwefel enthaltenen flüchtigen organischen Verbindungen und flüchtigen siliziumhaltigen und/oder flüchtigen schwefelhaltigen organischen Verbindungen; oder
(4) bei schwefelreichen Einsatzprodukten mit >100 ppm Schwefel, bei denen die Schwefelkomponente unter Crackbedingungen flüchtig wird, nur siliziumhaltigen flüchtigen organischen Verbindungen, höchstens jedoch 300 ppm der reinen siliziumhaltigen Verbindung; wobei in jedem Fall das Atomverhältnis von Silizium und Schwefel jeweils 5:1 bis 1:1 beträgt; und das Gemisch mit den Wärmeaustauschflächen, die Temperaturen im Bereich von 400 bis 1000 'C aufweisen, in Kontakt gebracht wird.

2. Verfahren nach Anspruch 1, dadurch gekennzeichnet, daß die Wärmeaustauschfläche die metallische Innenwand eines Rohrreaktors und/oder Wärmeaustauschers ist.

3. Verfahren nach Anspruch 1, dadurch gekennzeichnet, daß die Silizium und/oder Schwefel enthaltende Verbindung ausgewählt ist aus der Gruppe, die aus Trimethylsilylmercaptan, Dimethylsulfid, Bis-trimethylsilylsulfid und deren Gemischen untereinander und mit Tetramethylsilan besteht.

4. Verfahren nach Anspruch 1, dadurch gekennzeichnet, daß das zu crackende Einsatzprodukt 50 bis 400 ppm, vorzugsweise 50 bis 200 ppm der Additivformulierung enthält.

5. Verfahren nach Anspruch 1, dadurch gekennzeichnet, daß das gecrackte Endprodukt ausgewählt ist aus der Gruppe, die aus Alkenen wie Ethylen, Propylen, Butadien besteht.

6. Verfahren nach Anspruch 1, dadurch gekennzeichnet, daß die Wärmeaustauschflächen Temperaturen im Bereich von 800 bis 1000 °C aufweisen.

## Claims

1. A method for the preparation of thermally cracked products from hydrocarbons, with the simultaneous reduction of the coke deposits on the heat-exchange surfaces, characterised in that 20 to 1000 ppm of an additive formulation are added to the charge product to be cracked, which formulation is selected from among
(1) one or more volatile organic compounds containing silicon and sulphur;
(2) a mixture of organic silicon-containing volatile compounds and organic sulphur-containing volatile compounds;
(3) a mixture of volatile organic compounds containing silicon and sulphur and volatile silicon-containing and/or volatile sulphur-containing organic compounds; or
(4) in the case of sulphur-rich charge products with >100 ppm sulphur, in which the sulphur component becomes volatile under cracking conditions, only silicon-containing volatile organic compounds, but at most 300 ppm of the pure silicon-containing compound; with in each case the atomic ratio of silicon and sulphur each time being 5:1 to 1:1;
and the mixture is brought into contact with the heat-exchange surfaces, which are at temperatures in the range from 400 to 1000°C.

2. A method according to Claim 1, characterised in that the heat-exchange surface is the metal inner wall of a tubular reactor and/or heat-exchanger.

3. A method according to Claim 1, characterised in that the compound containing silicon and/or sulphur is selected from the group which consists of trimethylsilyl mercaptan, dimethyl sulphide, bis-trimethylsilyl sulphide and mixtures thereof with each other and with tetramethylsilane.

4. A method according to Claim 1, characterised in that the charge product to be cracked contains 50 to 400 ppm, preferably 50 to 200 ppm, of the additive formulation.

5. A method according to Claim 1, characterised in that the cracked end product is selected from the group consisting of alkenes such as ethylene, propylene and butadiene.

6. A method according to Claim 1, characterised in that the heat-exchange surfaces are at temperatures in the range from 800 to 1000°C.

## Revendications

1. Procédé pour fabriquer des produits thermiquement craqués à partir d'hydrocarbures en réduisant simultanément les dépôts de coke sur les surfaces d'échange thermique,
caractérisé en ce que 20 à 1000 ppm d'une formulation d'additif sont ajoutés au produit de départ à craquer, laquelle est choisie parmi
(1) un ou plusieurs composés organiques volatils contenant du silicium et du soufre ;
(2) un mélange de composés volatils organiques contenant du silicium et de composés volatils organiques contenant du soufre ;
(3) un mélange de composés organiques volatils contenant du silicium et du soufre et de composés organiques contenant du silicium volatils et/ou contenant du soufre volatils ; ou
(4) pour des produits de départ riches en soufre ayant une teneur en soufre supérieure à 100 ppm, dans lesquels le composant soufré est volatil dans les conditions de craquage, uniquement des composés organiques volatils contenant du silicium, au plus cependant 300 ppm du composé pur contenant du silicium ;
le rapport atomique du silicium et du soufre valant dans chaque cas de 5:1 à 1:1 et le mélange étant amené en contact avec les surfaces d'échange thermique, qui présentent des températures dans la plage de 400 à 1000°C.

2. Procédé selon la revendication 1,
caractérisé en ce que la surface d'échange thermique est la paroi interne métallique d'un réacteur tubulaire et/ou d'un échangeur thermique.

3. Procédé selon la revendication 1,
caractérisé en ce que le composé contenant du silicium et/ou du soufre est choisi dans le groupe qui est constitué du triméthylsilylmercaptan, du diméthylsulfure, du bis-triméthylsilylsulfure et de leurs mélanges et avec du tétraméthylsilane.

4. Procédé selon la revendication 1,
caractérisé en ce que le produit de départ à craquer contient de 50 à 400 ppm, avantageusement de 50 à 200 ppm, de la formulation d'additif.

5. Procédé selon la revendication 1,
caractérisé en ce que le produit final craqué est choisi dans le groupe qui est constitué d'alcènes, comme l'éthylène, le propylène, le butadiène.

6. Procédé selon la revendication 1,
caractérisé en ce que les surfaces d'échange thermique présentent des températures dans la plage de 800 à 1000°C.
